# EUROPÄISCHE PATENTSCHRIFT

(11) **EP 0 401 484 B1**
(45) Veröffentlichungstag und Bekanntmachung des Hinweises auf die Patenterteilung: **22.12.1993**
(21) Anmeldenummer: 90105643.2
(22) Anmeldetag: 24.03.1990
(51) Int. Cl.: G01N 21/90

(54) **Verfahren zum Staubfreihalten einer Vorrichtung zum Feststellen von Fremdteilchen in Fluiden**
Method for maintaining dustfree, an apparatus for detecting foreign particles in fluids
Procédé pour maintenir sans poussière un appareil de détection de particules étrangères dans des fluides

(30) Priorität: 06.04.1989 DE 3911112
(43) Veröffentlichungstag der Anmeldung: 12.12.1990
(73) Patentinhaber: Harro Höfliger Verpackungsmaschinen GmbH, 71573 Allmersbach im Tal (DE)
(72) Erfinder: Höfliger, Harro,, D-7151 Allmersbach im Tal (DE); Barth, Gerhard, Prof.Dr., D-7500 Karlsruhe 41 (DE)
(74) Vertreter: Lempert, Jost, Dipl.-Phys. Dr. rer.nat.

(56) Entgegenhaltungen:
- EP-A- 0 277 629
- AT-A- 282 230
- DE-A- 2 912 419

## Beschreibung

Die Erfindung betrifft ein Verfahren zum Staubfreihalten des Strahlengangs bei einer Vorrichtung zum Feststellen von Fremdteilchen in Fluiden, insbesondere in in Flaschen enthaltenen Infusionslösungen oder dergleichen, mit einer Bleuchtungs- und einer Detektionseinrichtung, durch deren Strahlengang das Fluid geführt wird sowie eine zur Durchführung des Verfahrens ausgebildete Vorrichtung.

Bei Vorrichtungen zum Feststellen von Fremdteilchen in Fluiden, insbesondere von mikroskopischen Fremdteilchen hat sich herausgestellt, daß in den Strahlengang fliegende Staubteilchen aus der Umgebungsluft das Meßergebnis verfälschen können, da sie ein Fehlersignal bewirken können, obwohl die gerade untersuchte Flasche bzw. das Fluid selbst fehlerfrei sind. Eine derartige Vorrichtung ist grundsätzlich aus der DE-OS 37 03 306 bekannt, deren Offenbarungsgehalt zum Gegenstand der vorliegenden Anmeldung gemacht wird und auf die verwiesen wird.

Der Erfindung liegt daher die Aufgabe zugrunde, ein Verfahren und eine Vorrichtung zu schaffen, durch welche störende Einflüsse von Staubteilchen in der das zu untersuchende Fluid umgebenden Luft vermindert bzw. ausgeschaltet und die Detektionssicherheit einer Vorrichtung zum Feststellen von Fremdteilchen in Fluiden erhöht werden kann.

Erfindungsgemäß sieht ein Verfahren zur Lösung dieser Aufgabe vor, daß Reinluft vom Strahlengang her in den Förderweg des Fluids geblasen wird. Zur Lösung der Aufgabe ist eine gattungsgemäße Vorrichtung dadurch gekennzeichnet, daß der Förderkanal des Fluids im Bereich der Beleuchtungs- und Detektionseinrichtung in Richtung des Strahlengangs eine Öffnung aufweist und daß vom Förderweg aus gesehen jenseits der Öffnung ein mit einem Filter versehenes Gebläse angeordnet ist.

Es wäre naheliegend, wie dies auch bei geschlossenen reinzuhaltenden Räumen der Fall ist, aus diesen die Luft abzusaugen, wobei die abgesaugte Luft aufgrund entstehenden Unterdrucks gegebenenfalls über durch einen Filter einströmende Luft ersetzt wird. Es hat sich aber gezeigt, daß eine derartige Ausgestaltung bei derartigen Vorrichtungen zum Feststellen von Fremdteilchen in Fluiden nicht verwirklichbar ist bzw. zumindestens keine optimale Staubfreiheit, insbesondere im Detektions-Strahlengang, durch den das Fluid geführt wird, bewirkt. Die Erfindung geht daher einen anderen Weg und sieht vor, daß Reinluft vom Strahlengang her in den Förderweg des zuzuführenden Fluides geblasen wird. Damit wird zuverlässig ausgeschlossen, daß durch die notwendigerweise am Einlaß und Auslaß des Förderwegs für das Fluid gegebenen Öffnungen, durch die auch Staub eindringen könnte, Staub ein- und durch den Förderweg bis in den Strahlengang hindurchströmt. Durch die Erfindung wird vielmehr der Staub daran gehindert in den Förderweg des Fluids einzudringen bzw., soweit Staub etwas eindringt oder durch Anhaften an den das Fluid enthaltenden Flaschen mit hineingefördert wird, so wird es durch die Reinluftströmung fortgerissen und vom Strahlengang fort zu dem Ein- und Auslaß des Fluids in bzw. aus seinem Förderweg herausgeblasen. In bevorzugter Ausgestaltung kann dabei vorgesehen sein, daß die Reinluft sowohl in Zuführ- als auch Fortführrichtung in den Förderweg geblasen wird. Hierbei kann durch geeignete Leitmaßnahmen, wie Leitbleche oder dergleichen, vorgesehen sein, daß insbesondere entgegen der Förderrichtung ein größerer Anteil der Reinluft geblasen wird, da die Gefahr des Eindringens von Staub an der Eintrittsstelle des Fluids größer ist als im Austrittbereich. Eine weitere Ausbildung sieht vor, daß die Reinluft von der Beobachtungsseite der Beleuchtungs- und Detektionseinrichtung her in den Förderweg geblasen wird. Eine andere Ausbildung sieht vor, daß die Reinluft von einem Bereich hinter der Detektionseinrichtung entgegen dem Strahlengang der Beleuchtungs- und Detektionseinrichtung zum Förderweg geblasen wird. Eine Weiterbildung der erfindungsgemäßen Vorrichtung zeichnet sich dadurch aus, daß das Gebläse mit Filter relativ zum Förderkanal jenseits der Detektionseinrichtungen angeordnet ist.

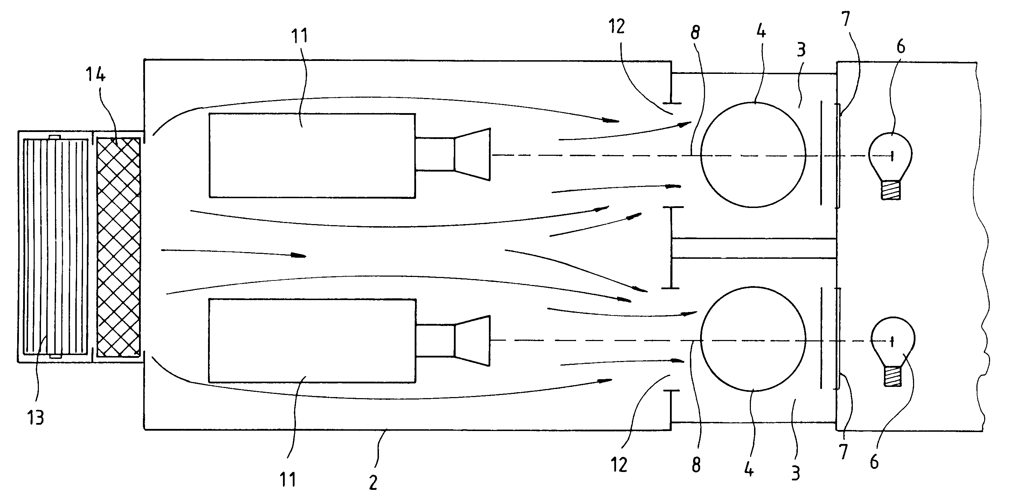

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnung im einzelnen erläutert ist. Dabei zeigt die einzige Figur
einen schematischen Horizontalschnitt durch eine bevorzugte Ausführungsform der erfindungsgemäßen Vorrichtung.

Bei der dargestellten erfindungsgemäßen Vorrichtung handelt es sich um eine Vorrichtung zum Feststellen von Fremdteilchen, insbesondere mikroskopischen Fremdteilchen in Fluiden, insbesondere in in Flaschen enthaltenen Infusionslösungen oder dergleichen, wie sie insbesondere aus der EP-A-277 629 an sich bekannt ist. Die Offenbarung dieser Druckschrift wird voll auch zum Gegenstand der vorliegenden Anmeldung gemacht. Die erfindungsgemäße Vorrichtung 1 weist ein Gehäuse 2 auf.

In dem Gehäuse 2 sind zwei senkrecht gerichtete Förderwege oder -kanäle 3 für in diesen zu fördernde Infusionsflaschen 4 vorgesehen, deren Inhalt untersucht werden kann. In der dargestellten Zeichnung befinden sich rechts der Förderwege 3 Lichtquellen 6, die von den Förderwegen 3 durch eine transparente Scheibe 7 getrennt sind. Weiterhin ist zwischen Lichtquelle 6 und Flasche 4 ein Polarisationsfilter sowie eine Blende bzw. ein Rahmen angeordnet, der insbesondere den mittleren Bereich der Flasche auf der optischen Achse 8 aus dem Lichtstrahl der Lampe 6 ausblendet. Diametral gegenüber der Lampe 6 relativ zur Flasche 4 befindet sich jeweils eine Kamera 11. Von dem jeweiligen Förderkanal 3 zur Kamera 11 ist eine Verbindungsöffnung 12 im Gehäuse 2 ausgebildet. Auf der den Förderwegen 3 abgewandten Seite der Kameras 11 ist am Gehäuse 1 ein Gebläse 13 mit einer zwischen diesem und dem Inneren des Gehäuses 2 angeordneten Filter 14 vorgesehen. Die Förderwege oder -kanäle 3 für die Infusionsflaschen 4 sind selbstverständlich an ihrem Einlaß und Auslaß offen, damit die Flaschen 4 in die Förderkanäle 3 hineingefördert werden können. Das Gebläse 13 saugt aus der Umgebung der Vorrichtung 1 Luft an und drückt diese durch das Filter 14 in das Innere des Gehäuses 2 hindurch, wobei die Luft durch das auswechselbare Filter 14 gereinigt wird. Die auf der Rückseite der Kameras 11 in das Innere des Gehäuses 2 hineingeförderte Luft strömt durch die Öffnungen 12 in die Förderkanäle 3 und entlang dieser Kanäle in Förderrichtung und entgegen der Förderrichtung der Flaschen 4 zu der Eintritts-und der Austrittsstelle der Flaschen, wo die in das Gehäuse 2 eingeblasene Luft aus dem Gehäuse wieder austritt. Hierdurch wird erreicht, daß der gesamte Strahlengang von den Kameras 11 her bis zu den Flaschen 4 und diese von gefilterter, gereinigter Luft umspült wird, so daß über den Ein-und den Auslaß für die Flaschen 4 Staubteilchen nicht eindringen können, bzw., wenn sie an den Flaschen anhaftend mit 35 in die Kanäle 3 gefördert werden, durch die Luftströmung von den Flaschen abgelöst und wieder aus den Kanälen 3 herausgespült werden. Insgesamt wird durch die erfindungsgemäße Anordnung eine vollständige Spülung des Gehäuseinneren und damit insbesondere ein Freihalten des Strahlenganges sowie der Oberflächen der zu untersuchenden Flaschen 4 im Untersuchungsbereich sichergestellt. Staubteilchen können also nicht zu einer Verfälschung des Meßergebnisses führen. Aufgrund der Freihaltung des Strahlengangs von Staubteilchen werden zuverlässig durch die erfindungsgemäße Prüfanordnung in der dargestellten Form lediglich Fremdteilchen in den Flaschen selbst erfaßt. Eine solche Reinigung des Strahlenganges ist insbesondere wichtig, wenn mikroskopische Fremdteilchen in der Größenordnung von wenigen zehn Mikrometern, die sich in den Flaschen 4 befinden, zuverlässig festgestellt werden sollen, um derartige Flaschen dann ausscheiden zu können.

## Patentansprüche

1. Verfahren zum Staubfreihalten des Strahlengangs bei einer Vorrichtung zum Feststellen von Fremdteilchen in Fluiden, insbesondere in in Flaschen enthaltenen Infusionslösungen oder dergleichen, mit einer Beleuchtungs- und einer Detektionseinrichtung, durch deren Strahlengang das Fluid geführt wird, dadurch gekennzeichnet, daß Staubfreie Reinluft vom Strahlengang her in den Förderweg des Fluids geblasen wird.

2. Verfahren nach Anspruch 1, dadurch gekennzeichnet, daß die Reinluft sowohl in Zuführ- als auch Fortführrichtung in den Förderweg geblasen wird.

3. Verfahren nach Anspruch 1 oder 2, dadurch gekennzeichnet, daß die Reinluft von der Beobachtungsseite der Beleuchtungs- und Detektionseinrichtung her in den Förderweg geblasen wird.

4. Verfahren nach einem der vorangehenden Ansprüche, dadurch gekennzeichnet, daß die Reinluft von einem Bereich hinter der Detektionseinrichtung entgegen dem Strahlengang der Beleuchtungs- und Detektionseinrichtung zum Förderweg geblasen wird.

5. Vorrichtung zum Feststellen von Fremdteilchen in Fluiden, insbesondere in in Flaschen enthaltenen Infusionslösungen oder dergleichen, mit einer Beleuchtungs- und einer Detektionseinrichtung, durch deren Strahlengang der Förderweg des Fluids führt, dadurch gekennzeichnet, daß der Förderkanal (3) des Fluids (Flasche 4) im Bereich der Beleuchtungs- und Detektionseinrichtung (6,12) in Richtung des Strahlengangs eine Öffnung (12) aufweist und daß vom Förderweg aus gesehen jenseits der Öffnung (12) ein mit einem Filter (14) versehenes Gebläse angeordnet ist.

6. Vorrichtung nach Anspruch 5, dadurch gekennzeichnet, daß das Gebläse (13) mit Filter relativ zum Förderkanal (3) jenseits der Detektionseinrichtungen (11) angeordnet ist.

## Claims

1. Method for keeping dust-free the optical path in an apparatus for detecting foreign particles in fluids, particularly in infusion solutions or the like contained in bottles, having an illuminating and a detecting device, through whose optical path is passed the fluid, characterized in that the dust-free pure air is blown from the optical path into the delivery path of the fluid.

2. Method according to claim 1, characterized in that the pure air is blown both in the supply and also the carrying away direction into the delivery path.

3. Method according to claim 1 or 2, characterized in that the pure air is blown into the delivery path from the observation side of the illuminating and detecting device.

4. Method according to one of the preceding claims, characterized in that the pure air is blown from an area behind the detecting device counter to the optical path of the illuminating and detecting device towards the delivery path.

5. Apparatus for detecting foreign particles in fluids, particularly in infusion solutions or the like contained in bottles, with an illuminating and a detecting device, through whose optical path is passed the delivery path of the fluid, characterized in that the delivery channel (3) of the fluid (bottle 4), in the vicinity of the illuminating and detecting device (6, 12) in the direction of the optical path has an opening (12) and that considered from the delivery path, a blower provided with a filter is placed on the other side of the opening (12).

6. Apparatus according to claim 5, characterized in that the blower (13) with the filter is positioned relative to the delivery channel (3) on the other side of the detecting devices (11).

## Revendications

1. Procédé pour maintenir exempt de poussière l'espace parcouru par les rayons lumineux d'un appareil de détection de particules étrangères dans les fluides, notamment dans les solutions infusées ou solutions semblables contenues dans des flacons, équipé d'un dispositif d'éclairement et de détection, à travers le parcours du faisceau lumineux desquels le fluide est conduit, caractérisé en ce que de l'air purifié exempt de poussière est insufflé dans l'espace parcouru par le faisceau lumineux vers le canal de convoyage du fluide.

2. Procédé selon la revendication 1, caractérisé en ce que l'air purifié est insufflé aussi bien dans les directions d'entrée que de sortie du canal de convoyage.

3. Procédé selon la revendication 1 ou 2, caractérisé en ce que l'air purifié est insufflé du côté d'observation du dispositif d'éclairement et de détection dans le canal de convoyage.

4. Procédé selon l'une des revendications précédentes caractérisé en ce que l'air purifié est insufflé d'une zone située derrière le dispositif de détection dans le sens contraire du faisceau lumineux entre les dispositifs d'éclairement et de détection vers le canal de convoyage.

5. Dispositif de détection de particules étrangères dans des fluides, notamment dans des solutions infusées ou solutions semblables contenues dans des flacons, équipé d'un dispositif d'éclairement et de détection, entre le trajet du faisceau lumineux desquels passe le canal de convoyage du fluide, caractérisé en ce que le canal de convoyage (3) du fluide (flacon 4) présente une ouverture (12) dans la zone des dispositifs d'éclairement et de détection (6, 11) dans la direction du faisceau des rayons lumineux et en ce qu'une soufflerie (13) est équipée d'un filtre (14) situé au-delà de l'ouverture (12) vue du canal de convoyage.

6. Dispositif selon la revendication 5, caractérisé en ce que la soufflerie (13) équipée du filtre est disposée au-delà du dispositif de détection (11) par rapport au canal de convoyage (3).
